# EUROPEAN PATENT SPECIFICATION

(11) **EP 3 471 664 B1**
(45) Date of publication and mention of the grant of the patent: **10.05.2023**
(21) Application number: 17814016.6
(22) Date of filing: 14.06.2017
(51) Int. Cl.: A61F 2/24

(54) **MITRAL REGURGITATION TREATMENT DEVICE**
VORRICHTUNG ZUR BEHANDLUNG VON MITRALKLAPPENINSUFFIZIENZ
DISPOSITIF DE TRAITEMENT DE LA RÉGURGITATION MITRALE

(30) Priority: 16.06.2016 US 201662351277 P; 03.11.2016 US 201615342808
(43) Date of publication of application: 24.04.2019
(73) Proprietor: Ma, Jianlu, Irvine, CA 92618 (US)
(72) Inventor: Ma, Jianlu, Irvine, CA 92618 (US)
(74) Representative: Prinz & Partner mbB
(86) International application number: PCT/US2017/037475
(87) International publication number: WO 2017/218671

(56) References cited:
- EP-A2- 2 254 514
- EP-B1- 2 254 514
- WO-A1-2014/110023
- US-A- 2 011 100
- US-A1- 2007 061 010
- US-A1- 2011 208 297
- US-A1- 2012 029 628
- US-A1- 2013 166 023
- US-A1- 2014 046 426
- US-A1- 2014 142 691
- US-A1- 2014 222 142
- US-B2- 9 023 098

## Description

### 1. Field of the Invention

The present invention relates to an aortic valve device. The device treats mitral regurgitation by implanting the device inside the aortic valve position and pushing the aortic curtain and/or anterior leaflet of the mitral valve towards the mitral valve.

### 2. Description of the Prior Art

The human heart has four chambers and four valves. The heart valves control the direction of blood flow. Fully functional heart valves ensure proper blood circulation is maintained during cardiac cycle. Heart valve regurgitation, or leakage, occurs when the leaflets of the heart valve fail to come fully into contact (coapt) due to disease, such as congenital, torn chordae tendineae, lengthened chordae tendineae, enlarged left ventricle, damaged papillary muscles, damaged valve structures by infections, degenerative processes, calcification of the leaflets, stretching of the annulus, increased distance between the papillary muscles, etc. Regardless of the cause, the regurgitation interferes with heart function since it allows blood to flow back through the valve in the wrong direction. Depending on the degree of regurgitation, this backflow can become a self-destructive influence on not only the function, but also on the cardiac geometry. Alternatively, abnormal cardiac geometry can also be a cause of regurgitation, and the two processes may "cooperate" to accelerate abnormal cardiac function. The direct consequence of heart valve regurgitation is the reduction of forward cardiac output. Depending on the severity of the leakage, the effectiveness of the heart to pump adequate blood flow into other parts of the body can be compromised.

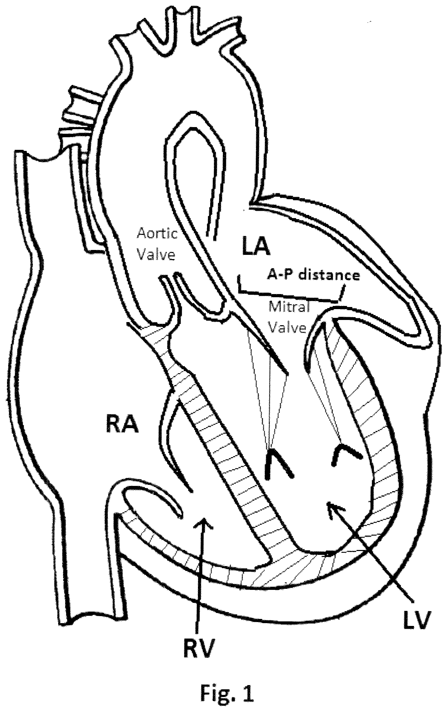

Referring to FIG. 1, the mitral valve is a dual-flap (bi-leaflet) valve in the heart that lies between the left atrium (LA) and the left ventricle (LV). During diastole, a normally-functioning mitral valve opens as a result of increased pressure from the left atrium as it fills with blood (preloading). As atrial pressure increases above that of the left ventricle, the mitral valve opens, facilitating the passive flow of blood into the left ventricle. Diastole ends with atrial contraction, which ejects the remainder of blood that is transferred from the left atrium to the left ventricle. The mitral valve closes at the end of atrial contraction to prevent a reversal of blood flow from left ventricle to left atrium. The human mitral valve is typically 4-6 cm² in opening area. There are two leaflets, the anterior leaflet and posterior leaflet, which cover the opening of the mitral valve. The opening of the mitral valve is surrounded by a fibrous ring called the mitral valve annulus. The two leaflets are attached circumferentially to the mitral valve annulus and can open and close by hinging from the annulus during cardiac cycle. In a normally-functioning mitral valve, the leaflets are connected to the papillary muscles in the left ventricle by chordae tendineae. When the left ventricle contracts, the intraventricular pressure forces the mitral valve to close, while chordae tendineae keep the two leaflets coapting (i.e., to prevent two valve leaflets from prolapsing into the left atrium and creating mitral regurgitation) and prevent the valve from opening in the wrong direction (thereby preventing blood from flowing back into the left atrium). Mitral valve regurgitation can be caused by failed coaptation of the native mitral leaflets. In other words, as shown in FIG. 1 , when the mitral leaflets failed to coapt, and blood flows back into the left atrium from ventricle during cardiac systole. FIG. 1 specifically shows the mitral valve with mitral regurgitation (during cardiac diastole) having a longer A-P distance.

Currently, the standard heart valve regurgitation treatment options include surgical repair/treatment and endovascular clipping. The standard surgical repair or replacement procedure requires open-heart surgery, use of cardio-pulmonary bypass, and stoppage of the heart. Because of the invasive nature of the surgical procedure, risks of death, stroke, bleeding, respiratory problems, renal problems, and other complications are significant enough to exclude many patients from surgical treatment.

In recent years, endovascular clipping techniques have been developed by several device companies. In this approach, an implantable clip made from biocompatible materials is inserted into the heart valve between the two leaflets to clip the middle portion of the two leaflets (mainly A2 and P2 leaflets) together to prevent the prolapse of the leaflets. However, some shortcomings have been uncovered in the practical application of endovascular clipping, such as difficulty of positioning, difficulty of removal once implanted incorrectly, recurrence of heart valve regurgitation, the need for multiple clips in one procedure, strict patient selection, etc.

EP 2 254 514 A2 discloses a stent valve having a generally tubular body portion wherein an area adjacent to a first end has a first stiffness and an area adjacent the second end has a second stiffness. A central region of the stent has a third stiffness that is less than the stiffness of the first and second ends. The central region of the stent has a structure that can be matched to a curved patient anatomical region.

In conclusion, there is a great need for developing a novel medical device to treat mitral regurgitation. None of the existing medical devices to date fully address this need. The present invention aims to provide physicians with a device which can avoid a traumatic surgical procedure, and instead provide a medical device that can be implanted through a catheter-based, less invasive procedure for mitral regurgitation treatment.

### SUMMARY OF THE INVENTION

According to the present invention, an aortic valve device is provided as defined in claim 1. The aortic valve device is suitable for treating mitral regurgitation. The device has a frame that has an annulus support, an aortic flange extending from one end of the annulus support, and a ventricular flange extending from another end of the annulus support, with the ventricular flange flared radially outwardly so that the ventricular flange gradually increases in diameter until it reaches a ventricular end. The frame further includes a tenting element that extends from a portion of the circumference of the ventricular end that is less than 90% of the circumference of the ventricular end, with the tenting element defining one or more cellular elements that are formed by struts that are connected to the ventricular end. The tenting element is located at a side of the circumference of the ventricular end that is positioned closer to a patient's aortic curtain when the frame is implanted in the aortic portion so that the tenting element pushes the structure of the mitral valve towards the posterior side of the mitral valve. The device also includes a set of leaflets sutured into the interior of the frame, with the leaflets replacing the valve function of the patient's native aortic valve.

Thus, the present disclosure provides a method and a device for treating mitral regurgitation. It treats mitral regurgitation by implanting the device inside the aortic valve and using the tenting element to push the aortic curtain and/or anterior leaflet of the mitral valve toward the mitral valve direction, thereby reducing the size of the mitral annulus (especially A-P distance), and improving the coaptation of the native mitral leaflets.

### BRIEF DESCRIPTION OF THE DRAWINGS

FIG. 1 illustrates a human heart showing an mitral valve that experiences mitral regurgitation.
FIG. 2 illustrates a human heart with the device implanted at the aortic position.
FIG. 3 is a perspective view of a device according to an embodiment.
FIG. 4 is a perspective view of the frame of the device of FIG. 3.
FIG. 5 is a side perspective view of the frame of FIG. 4.
FIG. 6 is a top plan view of the frame of FIG. 4.
FIG. 7 is a bottom plan view of the frame of FIG. 4.
FIG. 8 is a perspective view of a possible leaflet and skirt assembly that can be used with the device of FIG. 3.
FIGS. 9A-9C illustrate the delivery of the device of FIG. 3 using a transfemoral approach.
FIGS. 10A-1 0B illustrate the delivery of the device of FIG. 3 using a transapical approach.

### DETAILED DESCRIPTION OF PREFERRED EMBODIMENTS

The following detailed description is of the best presently contemplated embodiments of carrying out the invention. In recent years, several transcatheter aortic valve replacement devices (TAVI) have been developed and commercially available. These commercial available transcatheter aortic valves have shown some favorable clinical benefits and have been widely used throughout the world in treating patients with diseased aortic valves. Currently, the transcatheter aortic valve can be delivered transfemorally or transapically, or through other arteries in the body. The clinical evidence has shown that the transcatheter aortic valve replacement procedure is a safe and effective procedure.

The present disclosure provides a method and design for a mitral regurgitation treatment device 20 which treats mitral regurgitation by implanting the device inside the aortic valve and pushing the aortic curtain and/or anterior leaflet of the mitral valve toward the mitral valve direction via a tenting element 22 in the device to reduce the size of the mitral annulus, thereby improving the coaptation of the native mitral leaflets. The tenting element 22 in the device can also reduce the size of the mitral annulus (especially the A-P distance) by pushing/tenting the aortic curtain or anterior leaflet of the mitral valve, hence treating mitral valve regurgitation. The traditional aortic valve replacement procedure can be used in the method of the present disclosure to deliver the new device for mitral regurgitation treatment. Once the new device 20 is implanted in the aortic position, the tenting element 22 can push the aortic curtain/anterior leaflets /annulus of the mitral valve to reduce the A-P distance of the mitral valve and improve the coaptation of the mitral valve leaflets.

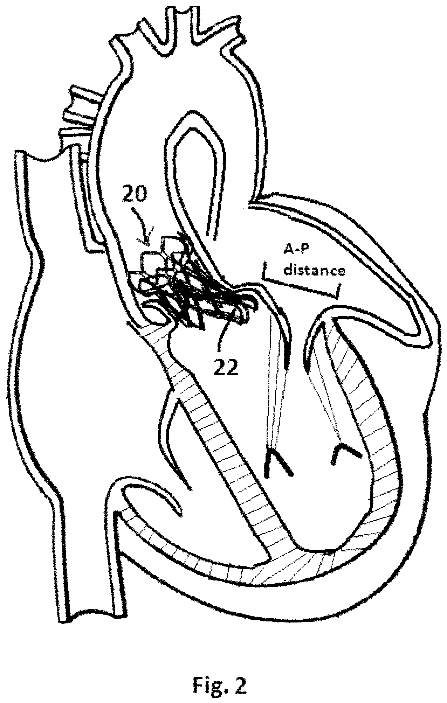

FIG. 2 illustrates a native mitral valve having a smaller A-P distance after the device 20 is implanted in the aortic valve position. The tenting element 22 of the device 20 pushes the anterior structure of the mitral valve towards the posterior side and reduces the A-P distance. During cardiac systole, the mitral valve leaflets can coapt properly (with reduced or no mitral regurgitation).

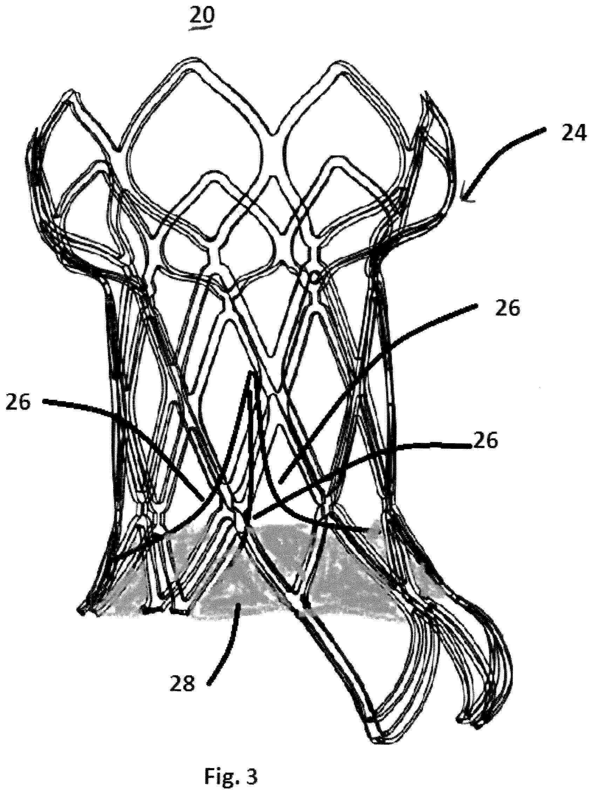

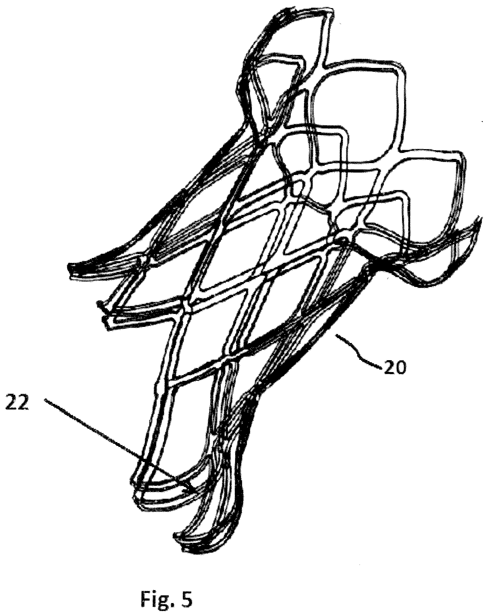

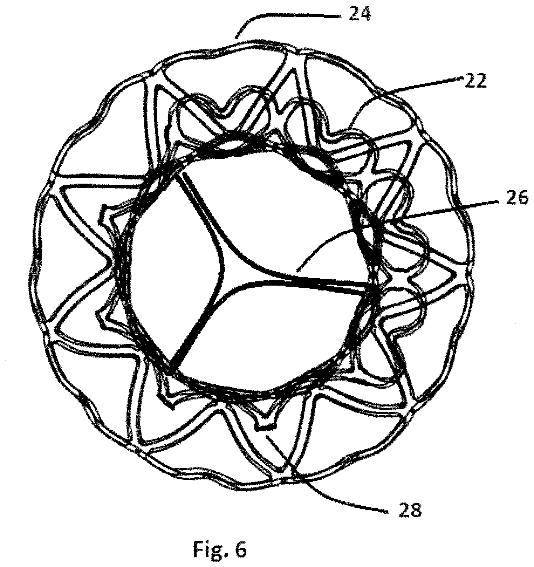

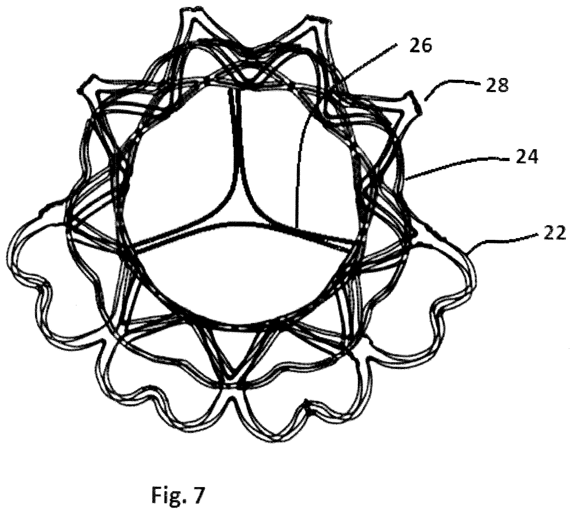

FIGS. 3-8 illustrate embodiments of the device 20 in greater detail. The device 20 includes a frame 24, a set of leaflets 26 sutured into the interior of the frame 24, and a skirt 28 that functions to prevent parivalvular leakage, to reduce trauma to the surrounding anatomy, and to promote tissue growth and healing.

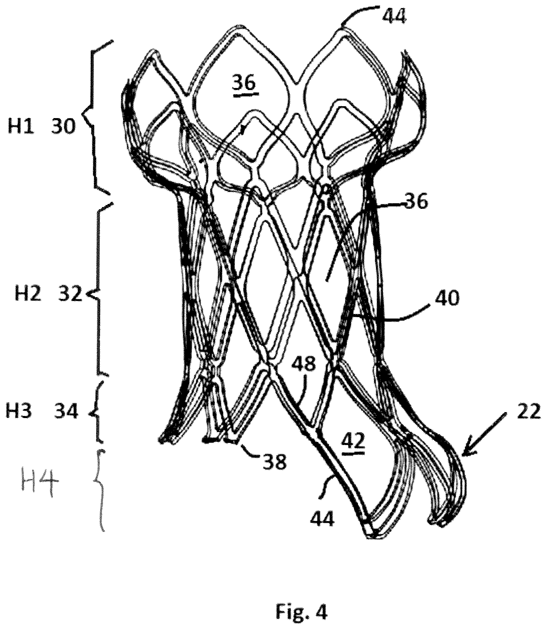

The frame 24 has an aortic flange 30, an annulus support 32 and a ventricular flange 34. The aortic flange 30, the annulus support 32 and the ventricular flange 34 can be made from either a Nitinol superelastic material or stainless steel, Co-Cr based alloy, Titanium and its alloys, and other self -expandable or balloon expandable biocompatible materials. Other polymer biocompatible materials can also be used to fabricate these components of the device 20. For example, the frame 24 can be laser cut from metal or polymer tubing. The cut structure would then go through shape setting, micro-blasting, and electro-polishing processes to achieve the desired profile/shape, as shown in FIG. 4. As an alternative, the frame 24 can also be fabricated from flat sheet, and then rolled to the desired shape.

The aortic flange 30 is adapted to be positioned in the aorta of the patient on the outflow side of the aortic valve, with a portion of the aortic flange 30 extending inside the aorta. The aortic flange 30 can be comprised of one annular row of cells 36 that are formed by interconnecting struts 40. The aortic flange 30 can have a surface area that is equal to or larger than the aortic annulus area.

The annulus support 32 functions as an anchoring feature, and can interact with the annulus. native leaflet(s), and other internal heart structures, or subvalvular structures, to provide the desired anchoring effect. See FIGS. 2 and 4. The annulus support 32 can define a generally cylindrical body that is made up of a plurality of cells 36 that are formed by interconnecting struts 40.

The ventricular flange 34 extends from the ventricular end of the annulus support 32, and can be flared radially outwardly so that the ventricular flange 34 can gradually increase in diameter until it reaches its ventricular end 38, where the diameter is greatest. The ventricular end 38 can be defined by the apices of the ventricular-most cells 36. The ventricular flange 34 can be comprised of the last annular row of struts 48 that define the ventricular-most cell 36 in the annulus support 32, with these struts 48 being flared outwardly. Radiopaque markers can be incorporated into ventricular flange 34 for visualization aid to facilitate positioning during the delivery of the device 20, and for follow-up post implantation. In use, the ventricular flange 34 and part of the height of the annulus support 32 can be covered by biocompatible polymer fabric, tissue or other biocompatible materials to provide a sealing effect around the device 20 and to promote tissue growth and speed up the healing effect.

The tenting element 22 extends from a portion of the circumference/perimeter of the ventricular end 38 and can be made or laser-cut from the same material as the rest of the frame 20. The tenting element 22 can be embodied in the form of one or more cellular elements 42 that are formed by struts 44 that are connected to the apices of the cells at the ventricular end 38. The tenting element 22 is located at the side of the circumference of the ventricular end 38 that is closer to the aortic curtain. Various forms of radiopaque markers, or coils, can be incorporated into the tenting element 22 for visualization, positioning, and directional positioning of the device 20 and the tenting element 22 during the procedure and during follow-up post implantation. The tenting element 22 extends along 1% to 90% of the circumference of the ventricular end 38. The cellular elements 42 preferably define a diameter that is greater than the outer diameter of the ventricular end 38, and can be curved outwardly. In the embodiment shown in FIGS. 3-7, the struts 44 that form the tenting element 22 extending radially outwardly from the ventricular end 38 in a concave manner such that the apices of the cellular elements 42 extend radially inwardly from the largest-diameter portion of the struts 44. The size of the cellular elements 42 can be smaller, larger, or the same as, the size as the cells 36 in the annulus support 32, depending on the amount of flexibility desired. The radiopaque markers can be incorporated into the annulus support 32, and/or into ventricular flange 34, and/or the tenting element 22, to help with the positioning of the device 20 during delivery. In addition, there can be more than one row of the cellular elements 42. For example, providing additional rows of cellular elements 42 would provide a tenting element 22 with different mechanical properties, for example, the tenting element 22 can either be more flexible than other portions of the frame, or stiffer than other portions by adjusting the dimensions of the cellular elements 42. In addition, the cellular elements 42 can be smaller in size. As another alternative, different rows of the tenting element 42 can have different sizes.

The width of each strut 40 can range from 0.2 mm to 2.5 mm, and the thickness of each strut 40 can range from 0.1 mm to 0.75 mm. The length of each cell 36 can be in the range from 2 mm to 25 mm. The number of cells 36 along the circumference of the annulus support 32 can range from 3 to 20.

FIG. 4 illustrates the typical dimensional or geometry range for each component of the device 20. The aortic flange 30 can either have a circular profile or a profile different from a full circle. Where the aortic flange 30 has a circular profile, the diameter of the aortic portion can be in the range from 12 mm to 50 mm. If the aortic flange 30 has a profile which is different from full circle, the long axis can be in the range from 20 mm to 50 mm. and the shorter axis can be in the range from 12 mm to 40 mm. In addition, the height H1 of the aortic flange 30 can range from 0.5 mm to 50 mm. At the upper aortic end of the aortic flange 30, each cell 36 that defines the aortic flange 30 has peaks and valleys, with a rounded non-traumatic tip 44 at each peak thereof. If needed, the aortic flange 30 can be either fully or partially covered by fabric or tissue material, or a combination of tissue and fabric materials. The aortic flange 30 can have barbs or spikes at the side that faces the outer surface to help engage the aorta wall if needed.

The annulus support 32 can have a height H2 in the range from 5 mm to 60 mm. The cross-sectional profile of the annulus support 32 can either be a full circular shape or a profile that is different from a circular shape. Where the annulus support 32 has a full circular profile, its diameter can be in the range from 12 mm to 50 mm. Where the annulus support 32 has a profile which is different from a circular shape, the long axis can be in the range from 15 mm to 50 mm, and the shorter axis can be in the range from 12 mm to 45 mm. The lower portion (i.e., closer to ventricular side) of the annulus support 32 can be either fully or partially covered by fabric or tissue material, or a combination of tissue and fabric materials. For example, one portion of the annulus support 32 can be covered by fabric, and another portion of the annulus support 32 can be covered by tissue, or vice versa. In use, the fabric material and tissue can either be sewn/connected together first, or sewn/connected individually onto the lower portion of the annulus support 32. The lower portion of the annulus support 32 can be covered either along one surface (i.e., internal or external surface), or along both surfaces (i.e., internal and external surface). At the bottom (ventricular) end of the annulus support 32, each cell 36 transitions into the ventricular flange 34. The ventricular flange 34 can have a height H3 in the range from 1 mm to 20 mm. The cross-sectional profile of the ventricular flange 34 can either be a full circular shape or a profile that is different from a circular shape. Where the ventricular flange 34 has a full circular profile, its diameter can be in the range from 12 mm to 60 mm. Where the ventricular flange 34 has a profile which is different from a circular shape, the long axis can be in the range from 15 mm to 60 mm, and the shorter axis can be in the range from 12 mm to 50 mm. The ventricular flange 34 can either be fully or partially covered by polymer or tissue material. The ventricular flange 34 can have a tapered configuration. For example, the end connects with annulus support 32 can have a diameter smaller than that of the ventricular end of the ventricular flange 34.

The tenting element 22 can have a height H4 in the range from 1 mm to 30 mm. Preferably, the height H4 is about 50% to 150% of the height H3, and about 10% to 70% of the height H2.

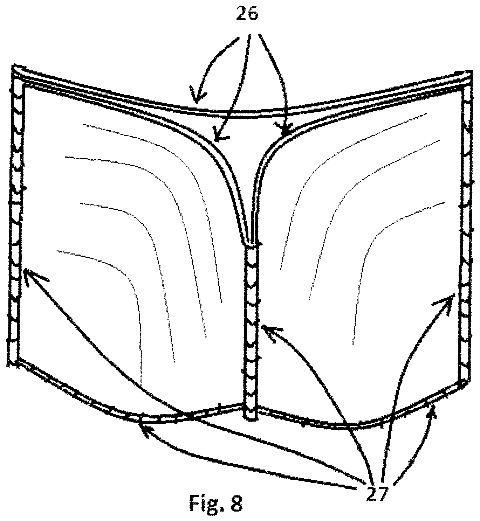

FIG. 8 shows an exemplary configuration of a leaflet and skirt assembly that can be used with the device 20, which can be a tri-leaflet design. Three leaflets 26 can be cut from fixed tissue, or polymer materials. The leaflets 26 can be sewn together by suturing along suture lines 27, and then sewn together with skirt materials 28 to generate the leaflet and skirt assembly. The leaflet and skirt assembly can then be integrated into the frame 24 by sewing or other mechanical means.

The leaflets 26 can be made from treated pericardial tissue, such as bovine or porcine tissue, or other biocompatible polymer materials. The leaflets 26 can also be made from thin wall biocompatible metallic element (such as stainless steel, Co-Cr based alloy, Nitinoi, Ta, and Ti etc.), or from biocompatible polymer material (such as polyisoprene, polybutadiene and their co-polymers, neoprene and nitrile rubbers, polyurethane elastomers, silicone rubbers, fluoroelastomers and fluorosolicone rubbers, polyesters, and PTFE, etc.). The leaflets can also be provided with a drug or bioagent coating to improve performance, prevent thrombus formation and promote endotheliolization. The leaflet(s) on the device 20 can also be treated or be provided with a surface layer/coating to prevent calcification.

The leaflets 26 can be integrated into the frame 24 by mechanical interweaving, suture sewing, and chemical, physical, or adhesive bonding methods. The leaflets 26 can also be coated with drug(s) or other bioagents to prevent the formation of clots in the heart. Anti-calcification materials can also be coated or provided on the surface to prevent calcification.

The skirt 28 can be made from either treated tissue or polymer materials, or the combination of these two materials.

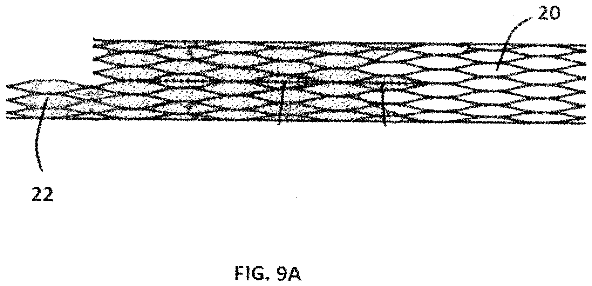

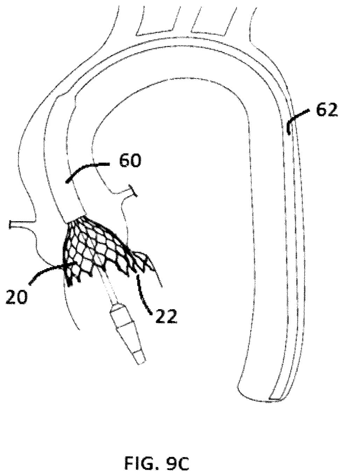

The device 20 can be delivered to the aortic position in a manner that is similar to that of the current transcatheter aortic valve (TAVI) replacement devices. The device 20 can be compacted into a low profile (see FIG. 9A) and loaded onto a delivery system that includes a delivery system sheath 60 and a delivery catheter 62, and then delivered to the target location by a non-invasive medical procedure, such as through the use of the delivery catheter 62 through transapical, or transfemoral, or transradial procedures, or through the carotid artery. The device 20 can be released from the delivery sheath 60 once it reaches the target implant site, and can expand to its normal (expanded) profile either by inflation of a balloon (for a balloon expandable frame 24) or by elastic energy stored in the device (for a device with a self-expandable frame 24). The device 20 can be pushed out of the delivery catheter 62, or the delivery catheter 62 can be withdrawn to release the device 20.

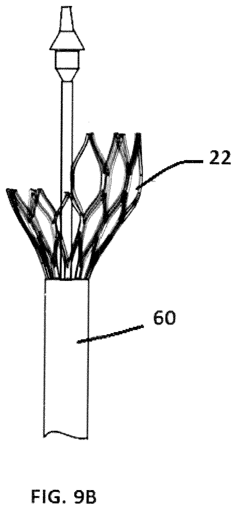

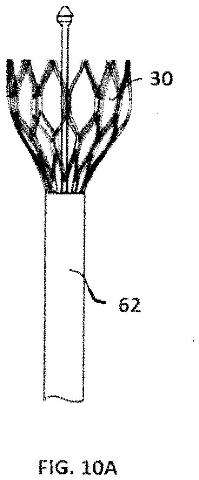

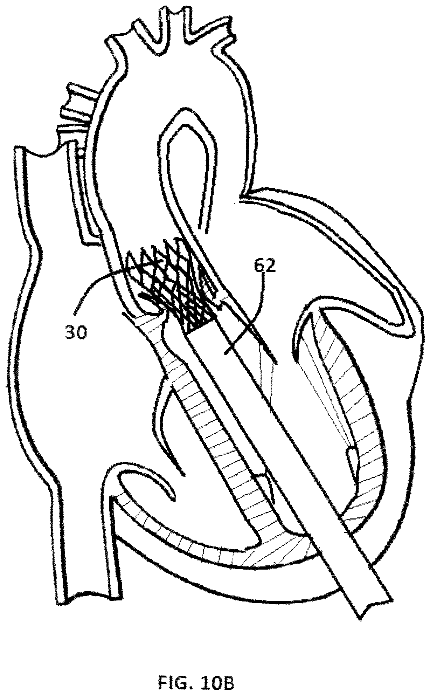

During the release of the device 20 from the delivery system, the components of the device 20 will be released out of the delivery system in sequence. For example, during transapical delivery, as shown in FIGS. 10A-10B, the aortic flange 30 will be deployed from the delivery sheath 60 first, then the annulus support 32, the ventricular flange 34, and then the tenting element 22, in that order. In contrast, during transfemoral delivery, as shown in FIGS. 9B-9C, the tenting element 22 will be deployed first, followed by the ventricular flange 34, the annulus support 32, and then aortic flange 30, in that order. The procedures can be performed under the guidance from x-ray and/or TEE, ICE, or other known imaging techniques. During the delivery, the direction of the delivery system and the device 20 will be controlled, so that when the device 20 is released form the delivery system, the tenting element 22 can be accurately located at the aortic curtain area to push the anterior mitral leaflet(s) toward mitral valve direction. The pushing action/force from the tenting element 22 can also reshape the mitral annulus (for example, reduce the A-P distance of the mitral valve, etc.), so that the native mitral leaflets can coapt and function better.

## Claims

1. An aortic valve device suitable for treating mitral regurgitation, comprising:
a frame (24) having an annulus support (32), an aortic flange (30) extending from one end of the annulus support (32), and a ventricular flange (34) extending from another end of the annulus support (32), the ventricular flange (34) flared radially outwardly so that the ventricular flange (34) gradually increases in diameter until it reaches a ventricular end (38);
a set of leaflets (26) sutured into the interior frame (24), with the leaflets assuming the valve function of the patient's native aortic valve,
**characterized in that**
the frame (24) further includes a tenting element (22) that extends from a portion of the circumference of the ventricular end (38) that is less than 90% of the circumference of the ventricular end (38), with the tenting element (22) defining one or more cellular elements (42) that are formed by struts (44) that are connected to the ventricular end (38) and with the tenting element (22) being located at a side of the circumference of the ventricular end (38) that is positioned closer to a patient's aortic curtain when the frame (24) is implanted in the aortic portion so that the tenting element (22) pushes the anterior structure of the mitral valve towards the posterior side of the mitral valve.

2. The device of claim 1, wherein the struts (44) that form the tenting element (22) extend radially outwardly from the ventricular end (38) in a concave manner such that apices of the cellular elements (42) extend radially inwardly.

3. The device of claim 1, wherein the annulus support (32) is defined by a plurality of cells (36), and wherein the cellular elements (42) of the tenting element (22) have a smaller size as the cells in the annulus support (32).

4. The device of claim 1, wherein the annulus support (32) is defined by a plurality of cells (36), and wherein the cellular elements (42) of the tenting element (22) have a larger size as the cells (36) in the annulus support (32).

5. The device of claim 1, wherein the annulus support (32) is defined by a plurality of cells (36), and wherein the cellular elements (42) of the tenting element (22) have the same size as the cells (36) in the annulus support (32).

6. The device of claim 1, wherein the ventricular flange (34) is defined by a plurality of cells (36), with the ventricular end (38) defined by the apices of the cells.

7. The device of claim 6, wherein the ventricular flange (34) and a part of the height of the annulus support (32) are covered by biocompatible polymer fabric, tissue or other biocompatible materials.

8. The device of claim 1, wherein the tenting element (22) has a height and the annulus support (32) has a height, and wherein the height of the tenting element (22) is about 10% to 70% of the height of the annulus support (32).

## Patentansprüche

1. Aortenklappenvorrichtung, die zur Behandlung von Mitralklappeninsuffizienz geeignet ist, mit:
einem Gerüst (24), das einen Ringträger (32), einen aortalen Flansch (30), der sich von einem Ende des Ringträgers (32) erstreckt, und einen ventrikulären Flansch (34) aufweist, der sich von einem anderen Ende des Ringträgers (32) erstreckt, wobei der ventrikuläre Flansch (34) radial nach außen aufgeweitet ist, so dass der ventrikuläre Flansch (34) allmählich im Durchmesser zunimmt, bis er ein ventrikuläres Ende (38) erreicht,
einem Satz Segel (26), die in das innere Gerüst (24) eingenäht sind, wobei die Segel die Klappenfunktion der natürlichen Aortenklappe des Patienten übernehmen,
**dadurch gekennzeichnet, dass**
das Gerüst (24) ferner ein zeltartiges Element (22) aufweist, das sich von einem Teil des Umfangs des ventrikulären Endes (38) erstreckt, der weniger als 90% des Umfangs des ventrikulären Endes (38) beträgt, wobei das zeltartige Element (22) ein oder mehrere zellenförmige Elemente (42) definiert, die durch Streben (44) gebildet sind, die mit dem ventrikulären Ende (38) verbunden sind, und wobei das zeltartige Element (22) an einer Seite des Umfangs des ventrikulären Endes (38) gelegen ist, die näher am aortalen Übergang eines Patienten positioniert ist, wenn das Gerüst (24) in den Aortenteil implantiert ist, so dass das zeltartige Element (22) die anteriore Struktur der Mitralklappe zur posterioren Seite der Mitralklappe hin schiebt.

2. Vorrichtung nach Anspruch 1, bei der sich die Streben (44), die das zeltartige Element (22) bilden, vom ventrikulären Ende (38) radial nach außen in einer konkaven Weise so erstrecken, dass sich die Spitzen der zellenförmigen Elemente (42) radial nach innen erstrecken.

3. Vorrichtung nach Anspruch 1, bei der der Ringträger (32) durch mehrere Zellen (36) definiert ist und bei der die zellenförmigen Elemente (42) des zeltartigen Elements (22) kleiner als die Zellen im Ringträger (32) sind.

4. Vorrichtung nach Anspruch 1, bei der der Ringträger (32) durch mehrere Zellen (36) definiert ist und bei der die zellenförmigen Elemente (42) des zeltartigen Elements (22) größer als die Zellen (36) im Ringträger (32) sind.

5. Vorrichtung nach Anspruch 1, bei der der Ringträger (32) durch mehrere Zellen (36) definiert ist und bei der die zellenförmigen Elemente (42) des zeltartigen Elements (22) die gleiche Größe wie die Zellen (36) im Ringträger (32) haben.

6. Vorrichtung nach Anspruch 1, bei der der ventrikuläre Flansch (34) durch mehrere Zellen (36) definiert ist, wobei das ventrikuläre Ende (38) durch die Spitzen der Zellen definiert ist.

7. Vorrichtung nach Anspruch 6, bei der der ventrikuläre Flansch (34) und ein Teil der Höhe des Ringträgers (32) mit biokompatiblem Polymergewebe, Gewebe oder anderen biokompatiblen Materialien bedeckt sind.

8. Vorrichtung nach Anspruch 1, bei der das zeltartige Element (22) eine Höhe und der Ringträger (32) eine Höhe aufweist und bei der die Höhe des zeltartigen Elements (22) etwa 10% bis 70% der Höhe des Ringträgers (32) beträgt.

## Revendications

1. Dispositif de valve aortique adapté au traitement de régurgitation mitrale, comprenant :
une armature (24) présentant un support annulaire (32), une bride aortique (30) qui s'étend depuis une extrémité du support annulaire (32), et une bride ventriculaire (34) qui s'étend depuis une autre extrémité du support annulaire (32), la bride ventriculaire (34) s'évasant radialement vers l'extérieur de sorte que la bride ventriculaire (34) s'agrandit progressivement en diamètre jusqu'à atteindre une extrémité ventriculaire (38) ;
un jeu de feuillets (26) suturé dans l'intérieur de l'armature (24), les feuillets assumant la fonction de valve de la valve aortique naturelle du patient,
**caractérisé en ce que**
l'armature (24) présente en outre un élément en tente (22) qui s'étend d'une partie de la circonférence de l'extrémité ventriculaire (38) qui est inférieure à 90% de la circonférence de l'extrémité ventriculaire (38), l'élément en tente (22) définissant un ou plusieurs éléments cellulaires (42) qui sont formés par des barrettes (44) reliées à l'extrémité ventriculaire (38), et l'élément en tente (22) étant agencé d'un côté de la circonférence de l'extrémité ventriculaire (38) qui est agencé plus proche de la séparation aortique du patient lorsque l'armature (24) est implantée dans la partie aortique, de sorte que l'élément en tente (22) pousse la structure antérieure de la valve mitrale vers le côté postérieur de la valve mitrale.

2. Dispositif selon la revendication 1, les barrettes (44) qui forment l'élément en tente (22) s'étendant depuis l'extrémité ventriculaire (38) radialement vers l'extérieur de manière concave, de sorte que les sommets des éléments cellulaires (42) s'étendent radialement vers l'intérieur.

3. Dispositif selon la revendication 1, le support annulaire (32) étant défini par une pluralité de cellules (36), et les éléments cellulaires (42) de l'élément en tente (22) présentant une taille inférieure à celle des cellules dans le support annulaire (32).

4. Dispositif selon la revendication 1, le support annulaire (32) étant défini par une pluralité de cellules (36), et les éléments cellulaires (42) de l'élément en tente (22) présentant une taille supérieure à celle des cellules (36) dans le support annulaire (32).

5. Dispositif selon la revendication 1, le support annulaire (32) étant défini par une pluralité de cellules (36), et les éléments cellulaires (42) de l'élément en tente (22) présentant la même taille que les cellules (36) dans le support annulaire (32).

6. Dispositif selon la revendication 1, la bride ventriculaire (34) étant définie par une pluralité de cellules (36), l'extrémité ventriculaire (38) étant définie par les sommets des cellules.

7. Dispositif selon la revendication 6, la bride ventriculaire (34) et une partie de la hauteur du support annulaire (32) étant recouvertes de tissu polymère biocompatible, de tissu ou d'autres matériaux biocompatibles.

8. Dispositif selon la revendication 1, l'élément en tente (22) présentant une hauteur et le support annulaire (32) présentant une hauteur, la hauteur de l'élément en tente (22) représentant environ 10% à 70% de la hauteur du support annulaire (32).
